(19) Europäisches Patentamt / European Patent Office / Office européen des brevets

(11) **EP 2 977 923 B1**

(12) **EUROPEAN PATENT SPECIFICATION**

(45) Date of publication and mention of the grant of the patent:
**22.01.2020 Bulletin 2020/04**

(21) Application number: **13879022.5**

(22) Date of filing: **19.03.2013**

(51) Int Cl.:
***G16B 15/30*** (2019.01)

(86) International application number:
**PCT/JP2013/057807**

(87) International publication number:
**WO 2014/147744 (25.09.2014 Gazette 2014/39)**

(54) **PROGRAM FOR DESIGNING COMPOUND, DEVICE FOR DESIGNING COMPOUND, AND METHOD FOR DESIGNING COMPOUND**

PROGRAMM ZUM DESIGN EINER VERBINDUNG, VORRICHTUNG ZUM DESIGN EINER VERBINDUNG UND VERFAHREN ZUM DESIGN EINER VERBINDUNG

PROGRAMME, DISPOSITIF ET PROCÉDÉ DE CONCEPTION D'UN COMPOSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.01.2016 Bulletin 2016/04**

(73) Proprietor: **Fujitsu Limited Kawasaki-shi, Kanagawa 211-8588 (JP)**

(72) Inventors:
• **MATSUMOTO, Shunji**
  **Kawasaki-shi**
  **Kanagawa 211-8588 (JP)**
• **TOMONAGA, Atsushi**
  **Kawasaki-shi**
  **Kanagawa 211-8588 (JP)**
• **KAMIYA, Nozomu**
  **Kawasaki-shi**
  **Kanagawa 211-8588 (JP)**
• **SUGIYAMA, Hajime**
  **Kawasaki-shi**
  **Kanagawa 211-8588 (JP)**

(74) Representative: **Hoffmann Eitle Patent- und Rechtsanwälte PartmbB Arabellastraße 30 81925 München (DE)**

(56) References cited:
**JP-A- H07 133 233     JP-A- 2005 187 374**

• **ROTSTEIN S H ET AL: "GENSTAR: A METHOD FOR DE NOVO DRUG DESIGN", JOURNAL OF COMPUTER-AIDED MOLECULAR DESIGN, ESCOM SCIENCE PUBLISHERS BV, XX, vol. 7, no. 1, 1 January 1993 (1993-01-01) , pages 23-43, XP008010867, ISSN: 0920-654X, DOI: 10.1007/BF00141573**
• **LUO Z ET AL: "RASSE: a new method for structure-based drug design", JOURNAL OF CHEMICAL INFORMATION AND COMPUTER SCIENCES, AMERICAN CHEMICAL SOCIETY, COLOMBUS,OHIO, US, vol. 36, no. 6, 1 November 1996 (1996-11-01), pages 1187-1194, XP002331826, ISSN: 0095-2338, DOI: 10.1021/CI950277W**
• **YOSHIHIKO NISHIBATA ET AL: "AUTOMATIC CREATION OF DRUG CANDIDATE STRUCTURES BASED ON RECEPTOR STRUCTURE STARTING POINT FOR ARTIFICIAL LEAD GENERATION", TETRAHEDRON, ELSEVIER SCIENCE PUBLISHERS, AMSTERDAM, NL, vol. 47, no. 43, 1 January 1991 (1991-01-01), pages 8985-8990, XP002911177, ISSN: 0040-4020, DOI: 10.1016/S0040-4020(01)86503-0**
• **SCHNEIDER G ET AL: "Computer-based de novo design of drug-like molecules", NATURE REVIEWS. DRUG DISCOVERY, NATURE PUBLISHING GROUP, GB, vol. 4, no. 8, 1 August 2005 (2005-08-01), pages 649-663, XP002435753, ISSN: 1474-1784, DOI: 10.1038/NRD1799**

EP 2 977 923 B1

**(Cont. next page)**

- DANIEL A. ERLANSON ET AL.: 'TETHERING: Fragment-Based Drug Discovery' 2004, XP008069432 Retrieved from the Internet: <URL:http:// www-nmr.cabm.rutgers.edu/academics/ biochem 694/ reading/Erlanson_etal_2004.pdf> [retrieved on 2013-04-04]

**Description**

TECHNICAL FIELD

The present invention relates to a compound design program, a compound design apparatus, and a compound design method.

BACKGROUND ART

Conventionally, there are techniques for designing a candidate medicine molecule on a computer. For example, a technique exists that includes disposing a fragment at an active site of a protein, further disposing a virtual single atom, and forming a bond if the interatomic distance and bond angle formed by the virtual single atom and the fragment or another virtual single atom are within stereochemically allowable ranges.

Patent Document 1: Japanese Laid-open Patent Publication No. 2005-187374

Rotstein S.H. et al. "GENSTAR: A METHOD FOR DE NOVO DRUG DESIGN", JOURNAL OF COMPUTER-AIDED MOLECULAR DESIGN, ESCOM SCIENCE PUBLISHERS BV, vol. 7, no. 1
Luo Z. et al., "RASSE: a new method for structure-based drug design", JOURNAL OF CHEMICAL INFORMATION AND COMPUTER SCIENCES, AMERICAN CHEMICAL SOCIETY, vol. 36, no. 6
Yoshihiko Nishibata et al., "AUTOMATIC CREATION OF DRUG CANDIDATE STRUCTURES BASED ON RECEPTOR STRUCTURE STARTING POINT FOR ARTIFICIAL LEAD GENERATION", TETRAHEDRON, ELSEVIER SCIENCE PUBLISHERS, vol. 47, no. 43

DISCLOSURE OF INVENTION

PROBLEM TO BE SOLVED BY THE INVENTION

However, a conventional technique often generates, as a linker that connects fragments, a chain linker, rather than a linker that includes a ring structure. Generation of a linker that includes a ring structure is desirable for a linker connecting fragments since a linker that includes a ring structure produces a thermodynamically advantageous interaction with protein as compared to the chain linker.

According to one aspect of the present invention, one object is to provide a compound design program, a compound apparatus, and a compound design method that can efficiently design a low-molecular compound that interacts with a protein.

MEANS FOR SOLVING PROBLEM

The above object is solved by the subject matter of the independent claims where advantageous embodiments are described in the dependent claims. Examples and technical descriptions of apparatuses, products and/or methods in the description and/or drawings which are not covered by the claims are presented not as embodiments of the invention but as background art or examples useful for understanding the invention. According to one aspect of an example, a compound design program, a compound apparatus, and a compound design method are proposed that dispose a virtual single atom at a position that does not collide with a protein, a fragment, or a virtual single atom disposed on a simulation space; form a bond when an interatomic distance and an angle formed by the disposed virtual single atom with an atom in the fragment or another virtual single atom are within predetermined ranges for bond length and bond angle; and execute a cyclization process when a set of three contiguous atoms including a virtual single atom forming a bond satisfies a cyclization condition under which a chemically proper ring may be formed when a virtual single atom is added to the three atoms.

EFFECT OF THE INVENTION

An aspect of the present invention achieves an effect in that a low-molecular compound that interacts with a protein can efficiently be designed.

BRIEF DESCRIPTION OF DRAWINGS

FIG. 1 is an explanatory view of an example of a compound design method according to an embodiment;
FIG. 2 is a block diagram depicting an example of hardware configuration of a compound design apparatus 100;
FIG. 3 is a block diagram of a functional configuration example of the compound design apparatus 100;
FIG. 4 is an explanatory view of a first cyclization condition;
FIG. 5 is an explanatory view of a second cyclization condition;
FIG. 6 is a flowchart of an example of a compound design process procedure of the compound design apparatus 100;
FIG. 7 is a flowchart of an example of a specific process procedure of a fragment selection process;
FIG. 8 is a flowchart of an example of a specific process procedure of a fragment disposal process;
FIG. 9 is a flowchart of an example of a specific process procedure of a molecular skeleton construction process;
FIG. 10 is an explanatory view of an example of a condition concerning whether virtual atom arrangement is possible;
FIG. 11 is an explanatory view of details of a condition (5) depicted in FIG. 10;

FIG. 12 is a flowchart of an example of a specific process procedure of a heteroatom replacement process;

FIG. 13 is an explanatory view (part 1) of a construction example of a molecular skeleton of a low-molecular compound;

FIG. 14 is an explanatory view (part 2) of a construction example of a molecular skeleton of a low-molecular compound.

## BEST MODE(S) FOR CARRYING OUT THE INVENTION

Embodiments of a compound design program, a compound design apparatus, and a compound design method will be described in detail with reference to the accompanying drawings.

(Example of Compound Design Method)

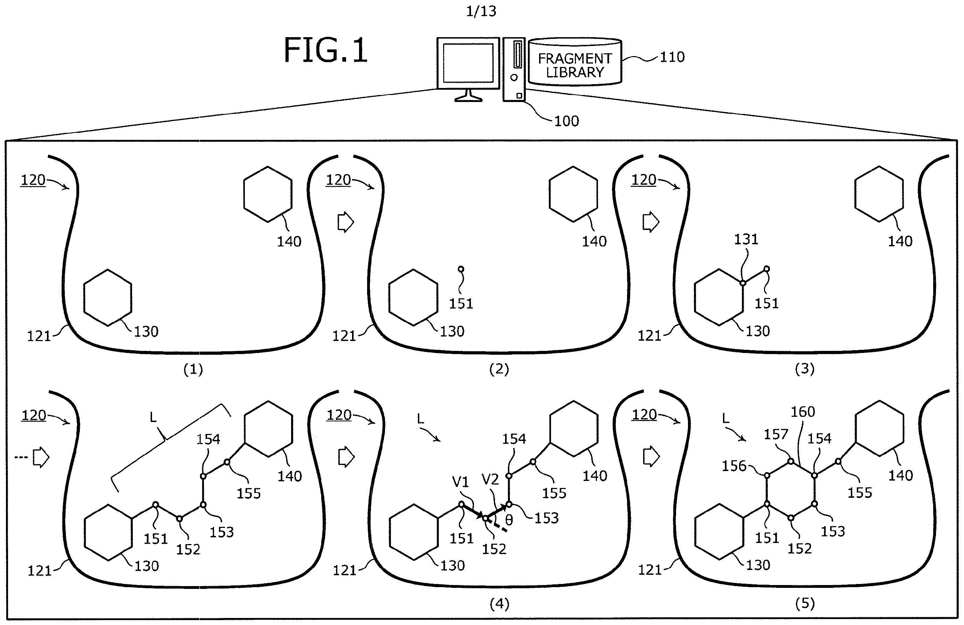

FIG. 1 is an explanatory view of an example of a compound design method according to an embodiment. In FIG. 1, a compound design apparatus 100 is a computer that has a fragment library 110 and designs low-molecular compounds that interact with protein. A low-molecular compound is a compound that interacts with a protein defined as a target (a target protein) to act as a drug.

For example, the body has a protein that decomposes sugar. If another protein bonds to this protein, the function of decomposing sugar may decrease, giving rise to a disease such as diabetes. The low-molecular compound bonds to an active site of the target protein to prohibit the target protein from bonding (reacting) with another protein, thereby acting as a drug.

The fragment library 110 includes, for example, a first library of real atoms (carbon, heteroatoms) and a second library in which all the real atoms of the first library are replaced with single virtual atoms (virtual single atoms). A fragment is a partial structure of a compound and refers to a molecule of one or more atoms, or an atom.

The first library is a database that stores in the form of a molecular structural formula (three-dimensional coordinates), fragments that frequently appear in physiologically active substances such as existing medicines and agrochemicals. Additionally, since amino acids are frequently applied as constituent elements of medicines, the first library may store side chains of 20 types of amino acids. The smallest partial structure stored in the first library is, for example, C (one carbon atom) of the alanine side-chain. The second library is a database that stores fragments obtained by replacing all the real atoms of the first library with single virtual atoms. The second library includes, for example, patterns of three-, four-, five-, six-, seven-membered rings, condensed rings, chain skeletons, and single atoms.

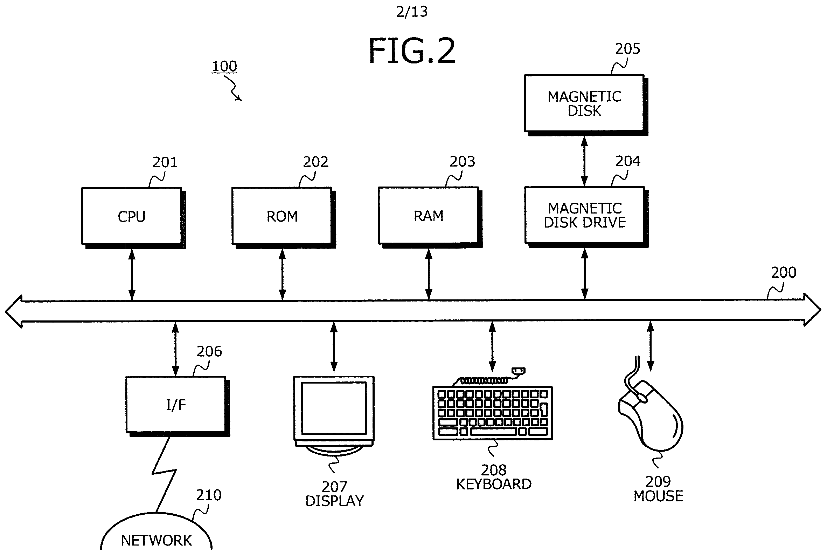

The fragment library 110 is realized by a storage apparatus such as a RAM 203 and a magnetic disk 205 as depicted in FIG. 2 described later, for example. The fragment library 110 may be included in another computer connected through a network 210 via an I/F 206 depicted in FIG. 2 described later, for example.

When a low-molecular compound is designed, for example, fragments are stably disposed at an active site of a target protein on a simulation space, and the fragments are connected by a partial structure called linker to construct a molecular skeleton of the low-molecular compound. As a linker to connect the fragments, the generation of a linker that includes a ring structure is desirable since a linker that includes a ring structure can produce a thermodynamically advantageous interaction with a protein as compared to a chain linker. Therefore, by designing a low-molecular compound having fragments connected by a linker that includes a ring structure, the probability of the low-molecular compound quickly and strongly bonding to the target protein rather than other proteins can be improved.

Thus, in the present embodiment, the compound design apparatus 100 executes a cyclization process when a set of three atoms on a linker connecting fragments disposed on a simulation space has the potential to make up a chemically proper ring if a virtual single atom is added to these atoms. In this way, the molecular skeleton of a low-molecular compound capable of producing a thermodynamically advantageous interaction with a protein is efficiently constructed. An example of a compound design process of the compound design apparatus 100 will be described hereinafter.

(1) The compound design apparatus 100 disposes fragments at an active site 121 of a protein 120 disposed on a simulation space. For example, the compound design apparatus 100 stably disposes at the active site 121 of the protein 120, multiple fragments selected from the fragment library 110.

Stable arrangement refers to entirely stable arrangement in terms of energy. A molecular dynamics calculation can be applied to search for arrangements. The molecular dynamics calculation can be achieved by applying a molecular dynamics (MD) method, a molecular mechanics (MM) method, a Monte Carlo (MC) method, etc.

In the example of FIG. 1, fragments 130, 140 are stably disposed at the active site 121 of the protein 120. The fragments 130, 140 are six-membered ring fragments obtained by replacing real atoms with virtual single atoms. For example, first, the compound design apparatus 100 disposes the fragments 130, 140 at the active site 121 of the protein 120.

The compound design apparatus 100 applies so-called fluctuation by MD calculation that uses force fields that repulse and attract each other when approaching and separating energetically, thereby stabilizing the fragments 130, 140 at the active site 102. To stably dispose the fragments 130, 140 at the active site 121, "positions" and "orientations" of the fragments 130, 140 are defined relative to the active site 121.

(2) The compound design apparatus 100 disposes a virtual single atom at a position that does not collide with the protein 120, the fragments 130, 140, or a virtual single atom disposed on the simulation space. In the example of FIG. 1, a virtual single atom 151 is disposed at a position that does not collide with the protein 120 or the fragments 130, 140.

(3) The compound design apparatus 100 forms a bond if the interatomic distance and angle formed by the disposed virtual single atom 151 with a virtual single atom in the fragments 130, 140 or another virtual single atom are within predetermined ranges for a bond length and a bond angle. The bond length in this case refers to a distance between atoms forming a bond. The bond angle is the angle of two bonds from a given atom.

The predetermined ranges of the bond length and the bond angle are respectively set in advance. The predetermined range of the bond length is set to a range of about 0.12 to 0.16 nm, for example. The predetermined range of the bond angle is set to a range of about 100 to 130 degrees, for example.

In the example depicted in FIG.1, the interatomic distance and angle formed by the virtual single atom 151 with a virtual single atom 131 in the fragment 130 are within the predetermined ranges for bond length and bond angle and therefore, a bond is formed between the virtual single atom 151 and the virtual single atom 131. The compound design apparatus 100 subsequently repeats the same processes as (2) and (3).

In this example, as a result of sequentially disposing virtual single atoms 152 to 155 on the simulation space, the fragments 130, 140 are connected by a linker L and the molecular skeleton of the low-molecular compound is constructed. However, the linker L is a chain linker without a ring structure at this point in time.

(4) The compound design apparatus 100 determines whether a set of three contiguous atoms including the virtual single atom forming a bond satisfies a cyclization condition under which a chemically proper ring may be formed if a virtual single atom is added to these atoms. The cyclization condition is defined by, for example, an angle formed between a vector from a first atom to a second atom and a vector from the second atom to a third atom in a set of the first, second, and third atoms contiguous through bonds. A set of the virtual single atom 151, the virtual single atom 152, and the virtual single atom 153 is taken as an example. In this case, for example, the compound design apparatus 100 determines that the cyclization condition is satisfied if an angle $\theta$ formed by a vector V1 from the virtual single atom 151 to the virtual single atom 152 and a vector V2 from the virtual single atom 152 to the virtual single atom 153 is a predetermined angle.

The predetermined angle is set in advance. For example, in the case of a six-membered ring, the predetermined angle is set to about 60 degrees. In this example, it is assumed that the angle $\theta$ formed by the vector V1 and the vector V2 is "$\theta \approx 60$ degrees." In this case, the compound design apparatus 100 determines that the virtual single atoms 151 to 153 satisfy the cyclization condition under which a chemically proper ring may be formed if a virtual single atom is added to these atoms.

(5) If it is determined that the cyclization condition is satisfied, the compound design apparatus 100 executes a cyclization process for a set of the three contiguous atoms including the virtual single atom forming a bond. The cyclization process in this case is a process of generating a ring by adding a virtual single atom to a set of three atoms on the simulation space. In the example of FIG. 1, virtual single atoms 156, 157 are added to virtual single atoms 151 to 154 to generate a hexagonal ring 160 on the linker L connecting the fragments 130, 140.

As described above, the compound design apparatus 100 can efficiently construct a molecular skeleton of a low-molecular compound with fragments connected by a linker that includes a ring structure. Therefore, a low-molecular compound capable of producing a thermodynamically advantageous interaction with a protein can be designed efficiently.

(Example of Hardware Configuration of Compound Design Apparatus 100)

FIG. 2 is a block diagram depicting an example of hardware configuration of the compound design apparatus 100. In FIG. 2, the compound design apparatus 100 has a central processing unit (CPU) 201, read-only memory (ROM) 202, random access memory (RAM) 203, a magnetic disk drive 204, a magnetic disk 205, an interface (I/F) 206, a display 207, a keyboard 208, and a mouse 209, respectively connected by a bus 200.

Here, the CPU 201 governs overall control of the compound design apparatus 100. The ROM 202 stores programs such as a boot program. The RAM 203 is used as a work area of the CPU 201. The magnetic disk drive 204, under the control of the CPU 201, controls the reading and writing of data to the magnetic disk 205. The magnetic disk 205 stores the data written thereto under the control of the magnetic disk drive 204.

The I/F 206 is connected to a network 210 such as a local area network (LAN), a wide area network (WAN), the Internet, etc. via a communications line, and is connected to other computers through the network 210. The I/F 206 administers an internal interface with the network 210, and controls the input and output of data with respect to other computers. A modem, LAN adapter, etc. can be used as the I/F 206, for example.

The display 207 displays data such as documents, images, and functional information, in addition to a cursor, icons, and toolboxes. A cathode ray tube (CRT), thin film transistor (TFT) liquid crystal display, a plasma display, and the like can be used as the display 207, for example.

The keyboard 208 has keys for inputting text, numerals, various instructions, etc. and performs the input of data. The keyboard 208 may be, for example, a touch panel input pad, a numeric key pad, and the like. The mouse 209 moves the cursor, selects ranges, moves windows, changes the size of windows, etc. The compound design apparatus 100 may further have an optical disk drive, a scanner, a printer, etc. in addition to the above components.

(Example of Functional Configuration of Compound Design Apparatus 100)

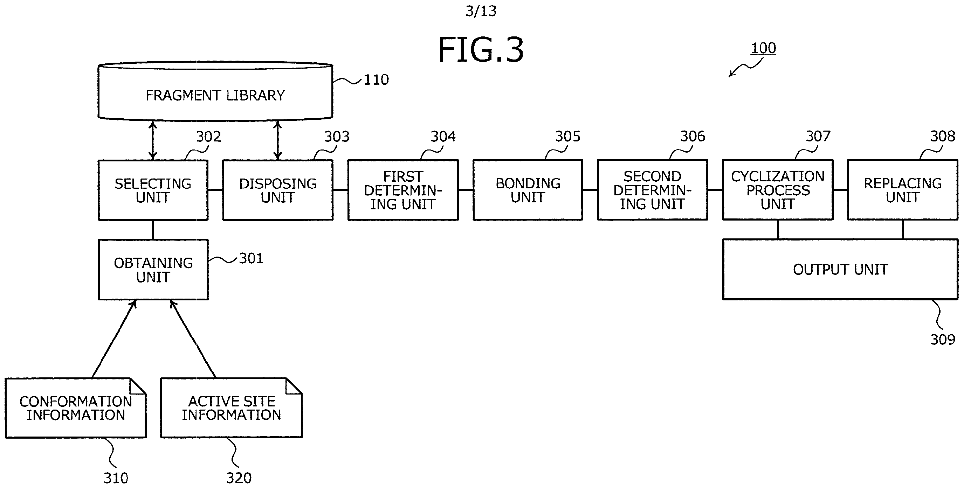

FIG. 3 is a block diagram of a functional configuration example of the compound design apparatus 100. In FIG. 3, the compound design apparatus 100 is configured to include an obtaining unit 301, a selecting unit 302, a disposing unit 303, a first determining unit 304, a bonding unit 305, a second determining unit 306, a cyclization process unit 307, a replacing unit 308, and an output unit 309. The obtaining unit 301 to the output unit 309 are functions acting as a control unit and, for example, the functions are realized by causing the CPU 201 to execute a program stored in a storage apparatus such as the ROM 202, the RAM 203, and the magnetic disk 205 depicted in FIG. 2, or by the I/F 206. Processing results of the functional units are stored to a storage apparatus such as the RAM 203 and the magnetic disk 205, for example.

The obtaining unit 301 has a function of obtaining conformation information 310 and active site information 320 of a protein defined as a target. The conformation information 310 is information representative of conformation of protein disposed on the simulation space. The active site information 320 is information representative of an active site of protein. The active site information 320 is information representative of a cube that includes an active site of protein disposed on the simulation space, for example.

For example, the obtaining unit 301 obtains the conformation information 310 and the active site information 320 of protein according to operational input from a user using the keyboard 208 and the mouse 209 depicted in FIG. 2. For instance, the obtaining unit 301 may obtain the conformation information 310 and the active site information 320 of protein from another computer via the network 210.

The selecting unit 302 has a function of selecting a fragment from the fragment library 110. For example, the selecting unit 302 selects a fragment having a number of atoms equal to or greater than a predetermined number N or a volume equal to or greater than a predetermined volume V and subsequently selects a fragment having a number of atoms less than the predetermined number N or a volume less than the predetermined volume V.

The number of atoms of a fragment is the total number of atoms making up the fragment. The volume of a fragment is a volume of a minimum space surrounding the fragment, for example. The numbers of atoms and the volumes of fragments are stored in the fragment library 110, for example. The predetermined number N and the predetermined volume V are set in advance and stored in a storage apparatus such as the ROM 202, the RAM 203, and the magnetic disk 205, for example.

The selecting unit 302 may select a fragment from the fragment library 110 according to operational input from a user using the keyboard 208 and the mouse 209. The fragment to be selected may be either a fragment stored in the first library, i.e., a real fragment, or a fragment stored in the second library, i.e., a fragment obtained by abstracting a real atom as a virtual single atom.

In the following description, a fragment having a number of atoms equal to or greater than the predetermined number N or having a volume equal to or greater than the predetermined volume V is referred to as a "large fragment" and a fragment having a number of atoms less than the predetermined number N or having a volume less than the predetermined volume V may be referred to as a "small fragment."

The disposing unit 303 has a function of disposing at an active site of a protein disposed on the simulation space, a fragment selected by the selecting unit 302. For example, the disposing unit 303 disposes a fragment at an active site of a protein such that an energetically stable arrangement overall is achieved by performing a disposal position search by using a molecular dynamics calculation (e.g., MD calculation) based on the conformation information 310 and the active site information 320 of the protein. As a result, the fragment can be disposed stably at the active site of the protein.

The disposing unit 303 has a function of disposing a virtual single atom at the active site of the protein disposed on the simulation space. For example, the disposing unit 303 disposes a virtual single atom at a position that does not collide with the protein, the fragments, or the other virtual single atoms disposed on the simulation space.

The first determining unit 304 has a function of determining the bondability between fragments, between a fragment and a virtual single atom, and between virtual single atoms disposed on the simulation space. For example, the first determining unit 304 determines that bonding is possible if an interatomic distance and an angle formed by the disposed virtual single atom with at least an already disposed fragment or another virtual single atom are within the predetermined ranges for bond length and bond angle. For example, the predetermined ranges of bond length and bond angle are respectively set in advance and stored in a storage apparatus such as the ROM 202, the RAM 203, and the magnetic disk 205.

The bonding unit 305 has a function of forming a bond between fragments, between a fragment and a virtual single atom, and between virtual single atoms disposed on the simulation space based on a determination result determined by the first determining unit 304. For example, the bonding unit 305 forms a bond between fragments, between a fragment and a virtual single atom, and between virtual single atoms determined by the first determining unit 304, to be bondable.

A series of processes by the disposing unit 303, the first determining unit 304, and the bonding unit 305 is repeatedly executed until the fragments selected by the selecting unit 302 are connected to each other by a linker to construct the molecular skeleton of the low-molecular compound, for example.

The second determining unit 306 has a function of determining whether a set of three contiguous atoms including the virtual single atom having a bond formed by the bonding unit 305 satisfies the cyclization condition under which a chemically proper ring may be formed if a virtual single atom is added to these atoms. For example, if an angle formed between a vector from the first atom to the second atom and a vector from the second atom to the third atom in a set of the three atoms is a predetermined angle $\alpha$, the second determining unit 306 may determine that the atoms satisfy the cyclization condition under which a chemically proper ring may be formed.

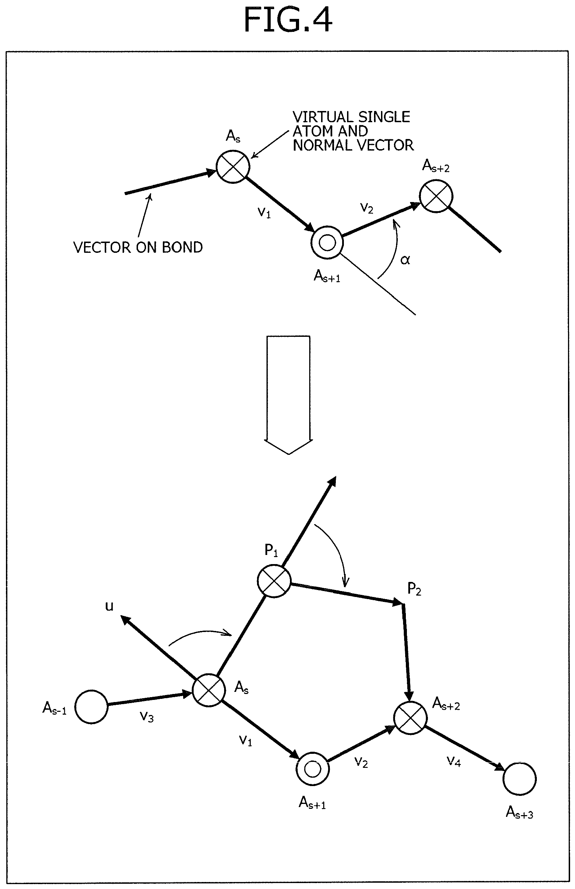

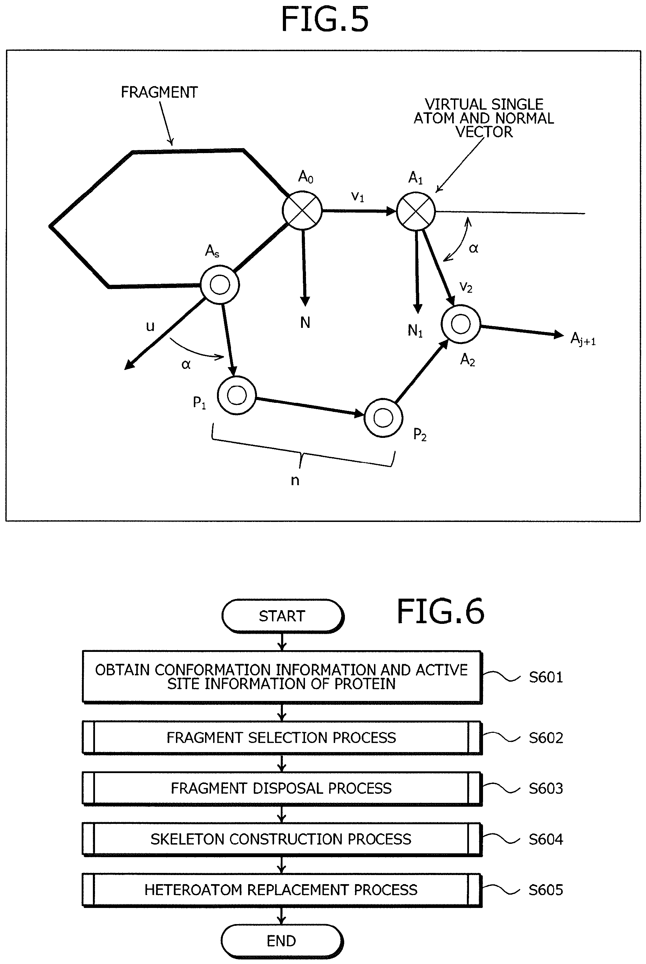

For example, the predetermined angle $\alpha$ is set in advance and stored in a storage apparatus such as the ROM 202, the RAM 203, and the magnetic disk 205. For example, the second determining unit 306 determines if a set of three atoms satisfies a first cyclization condition or a second cyclization condition described later. The first cyclization condition and the second cyclization condition will be described in detail later with reference to FIGs. 4 and 5.

The cyclization process unit 307 has a function of executing a cyclization process of generating a ring by adding a virtual single atom to a set of three atoms determined by the second determining unit 306 as satisfying the cyclization condition. For example, the cyclization process unit 307 disposes virtual single atoms at positions that may form a chemically proper ring with three atoms on the simulation space and forms bonds with these virtual single atoms to generate a ring. Specific process details of the cyclization process will be described later with reference to FIGs. 4 and 5.

The cyclization process unit 307 may terminate the cyclization process if the number of virtual single atoms in a portion connecting a generated ring to a fragment and/or a portion connecting generated rings to each other is equal to or less than a predetermined number $\beta$. For example, the predetermined number $\beta$ is set in advance and stored in the storage apparatus such as the ROM 202, the RAM 203, and the magnetic disk 205.

For example, the predetermined number $\beta$ is set to a value at which it can be determined that the degree of freedom of atoms in a low-molecular compound is reduced to a level facilitating interaction with protein if the number of virtual single atoms in a portion (chain portion) connecting a ring to a fragment and/or a portion (chain portion) connecting rings to each other is equal to or less than the predetermined number $\beta$. As a result, if the degree of freedom of atoms in a low-molecular compound is reduced to a level facilitating interaction with protein, the cyclization process can be terminated.

The replacing unit 308 has a function of replacing a virtual single atom with a heteroatom or a carbon atom. For example, the replacing unit 308 determines whether replacement with a heteroatom is effective based on increase/decrease in the energy value of electrostatic interaction and replaces a virtual single atom with a heteroatom based on the result. For example, the replacing unit 308 replaces the virtual single atoms in the molecular skeleton that includes the ring generated by the cyclization process unit 307 with a heteroatom one at a time and determines heteroatom replacement if the energy value for electrostatic interaction is reduced after the replacement.

The output unit 309 has a function of outputting molecular skeleton information representative of a molecular skeleton of a low-molecular compound that has fragments connected to each other by a linker that includes the ring generated by the cyclization process unit 307. The output unit 309 may output low-molecular compound information representative of a molecular skeleton after replacement in which a virtual single atom included in the molecular skeleton of the low-molecular compound is replaced with a heteroatom or a carbon atom by the replacing unit 308.

Configuration may be such that the compound design apparatus 100 does not have the replacing unit 308. In this case, another computer may execute a process of replacing with a heteroatom or a carbon atom, a virtual single atom included in the molecular skeleton of the low-molecular compound subjected to the cyclization process by the cyclization process unit 307.

(Specific Example of Cyclization Condition)

A specific example of the cyclization condition under which a chemically proper ring may be formed will be described with reference to FIGs. 4 and 5.

<First Cyclization Condition>

FIG. 4 is an explanatory view of a first cyclization condition. In FIG. 4, $A_s$, $A_{s+1}$, and $A_{s+2}$ represent three contiguous virtual single atoms disposed on the simulation space. In this case, a vector is drawn on a bond between virtual single atoms, and a start point $A_s$ and an end point $A_{s+2}$ are defined as points at which a direction of a normal vector formed by two vectors is reversed.

A vector $v_1$ is a vector from the virtual single atom $A_s$ to the virtual single atom $A_{s+1}$. The vector $v_1$ can be expressed by using Equation (1), for example. It is noted that $v_s$ is a vector from the origin to the virtual single atom $A_s$. Additionally, $v_{s+1}$ is a vector from the origin to the virtual single atom $A_{s+1}$. A vector $v_2$ is a vector from the virtual single atom $A_{s+1}$ to the virtual single atom $A_{s+2}$. The vector $v_2$ can be expressed by using Equation (2), for example. It is noted that $v_{s+2}$ is a vector from the origin to the virtual single atom $A_{s+2}$.

$$v_1 = v_{s+1} - v_s \qquad (1)$$

$$v_2 = v_{s+2} - v_{s+1} \qquad (2)$$

The second determining unit 306 determines that a pentagonal ring (m=5) may be generated when an angle $\alpha$ formed by the vector $v_1$ and the vector $v_2$ is "$\alpha \approx 72$ degrees." For example, if the angle $\alpha$ is within a range of about "71 degrees$<\alpha<$73 degrees," the second determining unit 306 determines that a pentagonal ring may be generated.

The second determining unit 306 determines that a hexagonal ring (m=6) may be generated when the angle $\alpha$ formed by the vector $v_1$ and the vector $v_2$ is "$\alpha \approx 60$ degrees." For example, if the angle $\alpha$ is within a range of about "59 degrees$<\alpha<$61 degrees," the second determining unit 306 determines that a hexagonal ring may be generated.

The second determining unit 306 determines that a heptagonal ring (m=7) may be generated when the angle $\alpha$ formed by the vector $v_1$ and the vector $v_2$ is "$\alpha \approx 51$ degrees." For example, if the angle $\alpha$ is within a range of about "51 degrees$<\alpha<$52 degrees," the second determining unit 306 determines that a heptagonal ring may be generated.

In FIG. 4, a cross mark (screw head) and a circle mark (screw tip) added into a circle representative of a virtual single atom indicates the direction of a normal vector. If the cross mark is added into a circle, the angle $\alpha$ that is identified is formed by the vector $v_1$ and the vector $v_2$ in clockwise rotation. In contrast, if the circle mark is added into a circle, the angle $\alpha$ that is identified is formed by the vector $v_1$ and the vector $v_2$ in counterclockwise rotation.

If it is determined that a polygonal ring with m vertices may be formed, the second determining unit 306 determines whether an angle formed by a vector $v_3$ is within a predetermined range $\gamma$ while an angle formed by a vector $v_4$ is within the predetermined range $\gamma$, relative to a $v_1$-$v_2$ plane. For example, the predetermined range $\gamma$ is set in advance and stored in the storage apparatus such as the ROM 202, the RAM 203, and the magnetic disk 205.

The vector $v_3$ is a vector from a virtual single atom $A_{s-1}$ to the virtual single atom $A_s$. The vector $v_3$ can be expressed by using Equation (3), for example. It is noted that $v_{s-1}$ is a vector from the origin to the virtual single atom $A_{s-1}$. The vector $v_4$ is a vector from the virtual single atom $A_{s+2}$ to a virtual single atom $A_{s+3}$. The vector $v_4$ can be expressed by using Equation (4), for example. It is noted that $v_{s+3}$ is a vector from the origin to the virtual single atom $A_{s+3}$.

$$v_3 = v_s - v_{s-1} \qquad (3)$$

$$v_4 = v_{s+3} - v_{s+2} \qquad (4)$$

If the angle formed by the vector $v_3$ is within the predetermined range $\gamma$ while the angle formed by the vector $v_4$ is within the predetermined range $\gamma$, the second determining unit 306 sets "U=-v1" and generates n vertices Pi (n=m-3) while rotating a vector u from the start point $A_s$ around an axis $N_s$ by $\alpha$ degrees. The axis $N_s$ is an axis extending in the same direction as the normal vector at the start point $A_s$.

If a distance $|P_n - A_{s+2}|$ is within a predetermined range of bond length, the second determining unit 306 determines that a set of the three contiguous virtual single atoms $A_s$, $A_{s+1}$, and $A_{s+2}$ satisfies the first cyclization condition. In this case, the cyclization process unit 307 executes the cyclization process of generating a ring for the set of the three contiguous virtual single atoms $A_s$, $A_{s+1}$, and $A_{s+2}$. For example, the cyclization process unit 307 forms bonds between the virtual single atoms $A_s$ and $P_1$, $P_i$ and $P_j$ (i>1, i<j, j<n), Pn and $A_{s+2}$ to generate a polygonal ring with m vertices.

For instance, it is assumed that a pentagonal ring (m=5) is generated. In this case, the cyclization process unit 307 sets "U=-v1" and disposes a virtual single atom $P_1$ at a vertex $P_1$ obtained by rotating the vector u from the start point $A_s$ around the axis $N_s$ by 72 degrees. The cyclization process unit 307 then bonds the virtual single atom $A_s$ to the virtual single atom $P_1$.

Subsequently, the cyclization process unit 307 disposes a virtual single atom $P_2$ at a vertex $P_2$ obtained by rotating the vector u from the vertex $P_1$ around the axis $N_s$ by 72 degrees. It is noted that this vector u is a vector from the start point $A_s$ to the vertex $P_1$. The cyclization process unit 307 then bonds the virtual single atom $P_1$ to the virtual single atom $P_2$ as well as the virtual single atom $P_2$ to the virtual single atom $A_{s+2}$. As a result, the pentagonal ring can be generated by using a set of the three contiguous virtual single atoms $A_s$, $A_{s+1}$, and $A_{s+2}$.

<Second Cyclization Condition>

A second cyclization condition will be described. The second cyclization condition is a condition for determining whether a chemically proper ring may be formed by using a vertical single atom in a fragment disposed on the simulation space.

FIG. 5 is an explanatory view of the second cyclization condition. In FIG. 5, $A_s$ and $A_0$ represent vertical single atoms in a fragment disposed on the simulation space. $A_1$ and $A_2$ represent two contiguous vertical single atoms disposed on the simulation space.

The second determining unit 306 sets "$u=v_s-v_0$" and determines whether a normal vector N formed by the vector $v_1$ and the vector u is in the same direction as a normal vector $N_1$ formed by the vector $v_1$ and the vector $v_2$. In this case, the vector $v_s$ is a vector from the origin to the virtual single atom $A_s$. The vector $v_0$ is a vector from the origin to the virtual single atom $A_0$. The vector $v_1$ is a vector from the virtual single atom $A_0$ to the virtual single atom $A_1$. The vector $v_2$ is a vector from the virtual single atom $A_1$ to the virtual single atom $A_2$.

If the normal vector N is in the same direction as the normal vector $N_1$, the second determining unit 306 determines that a pentagonal ring (m=5) may be generated when the angle $\alpha$ formed by the vector $v_1$ and the vector $v_2$ is "$\alpha \approx 72 degrees$." If the normal vector N is in the same direction as the normal vector $N_1$, the second determining unit 306 determines that a hexagonal ring (m=6) may be generated when the angle $\alpha$ formed by the vector $v_1$ and the vector $v_2$ is "$\alpha \approx 60 degrees$." If the normal vector N is in the same direction as the normal vector $N_1$, the second determining unit 306 determines that a heptagonal ring (m=7) may be generated when the angle $\alpha$ formed by the vector $v_1$ and the vector $v_2$ is "$\alpha \approx 51 degrees$."

If it is determined that a polygonal ring with m vertices may be formed, the second determining unit 306 defines, as an end point, a point at which the normal vector is reversed after the vertex $A_2$ (the virtual single atom $A_2$). The second determining unit 306 generates n vertices Pi (n=m-4) while rotating the vector u from a vertex adjacent to the vertex $A_0$ (the virtual single atom $A_0$) on the fragment, around an axis -N by $\alpha$ degrees.

If a distance $|P_n-A_2|$ is within a predetermined range of bond length, the second determining unit 306 determines that a set of the three contiguous virtual single atoms $A_0$, $A_1$, and $A_2$ satisfies the second cyclization condition. In this case, the cyclization process unit 307 executes the cyclization process of generating a ring for the set of the three contiguous virtual single atoms $A_0$, $A_1$, and $A_2$. For example, the cyclization process unit 307 forms bonds between the virtual single atoms $A_s$ and $P_1$, $P_i$ and $P_j$ (i>1, i<j, j<n), Pn and $A_2$ to generate a polygonal ring with m vertices.

For instance, it is assumed that a hexagonal ring (m=6) is generated. In this case, the cyclization process unit 307 disposes virtual single atoms $P_1$, $P_2$ at vertices $P_1$, $P_2$ generated while rotating the vector u from the vertex $A_s$ adjacent to the vertex $A_0$ (the virtual single atom $A_0$) on the fragment, around the axis -N by $\alpha$ degrees. The cyclization process unit 307 then bonds the virtual single atom $A_s$ to the virtual single atom $P_1$, bonds the virtual single atom $P_1$ to the virtual single atom $P_2$, and bonds the virtual single atom $P_2$ to the virtual single atom $A_2$. As a result, a hexagonal ring can be generated by using the virtual single atoms $A_s$, $A_0$ in the fragment.

(Compound Design Process Procedure of Compound Design Apparatus 100)

A compound design process procedure of the compound design apparatus 100 will be described.

FIG. 6 is a flowchart of an example of the compound design process procedure of the compound design apparatus 100. In the flowchart depicted in FIG. 6, first, the compound design apparatus 100 obtains the conformation information 310 and the active site information 320 of a protein disposed on the simulation space (step S601).

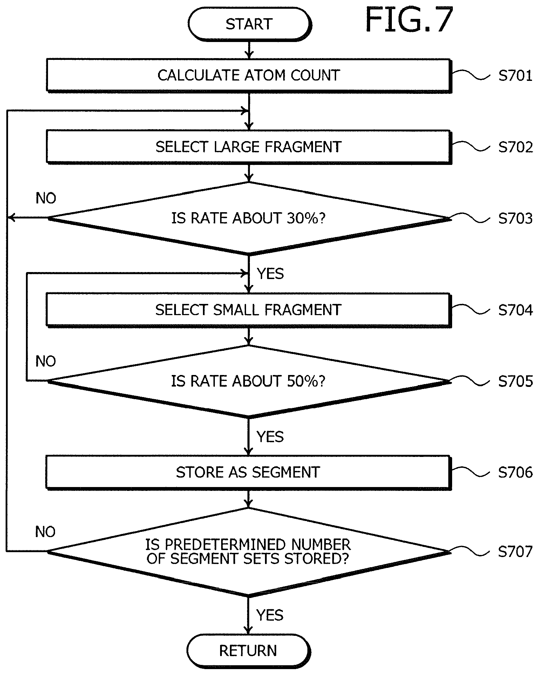

The compound design apparatus 100 executes a fragment selection process (step S602). The fragment selection process is a process of selecting from the fragment library 110, a fragment set of fragments to be disposed. A specific process procedure of the fragment selection process will be described later with reference to FIG. 7.

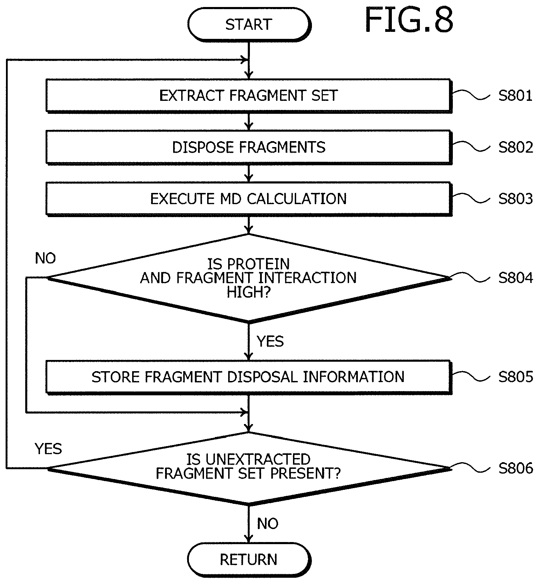

The compound design apparatus 100 executes a fragment disposal process (step S603). The fragment disposal process is a process of disposing the fragments selected in the fragment selection process at an active site of the protein disposed on the simulation space. A specific process procedure of the fragment disposal process will be described later with reference to FIG. 8.

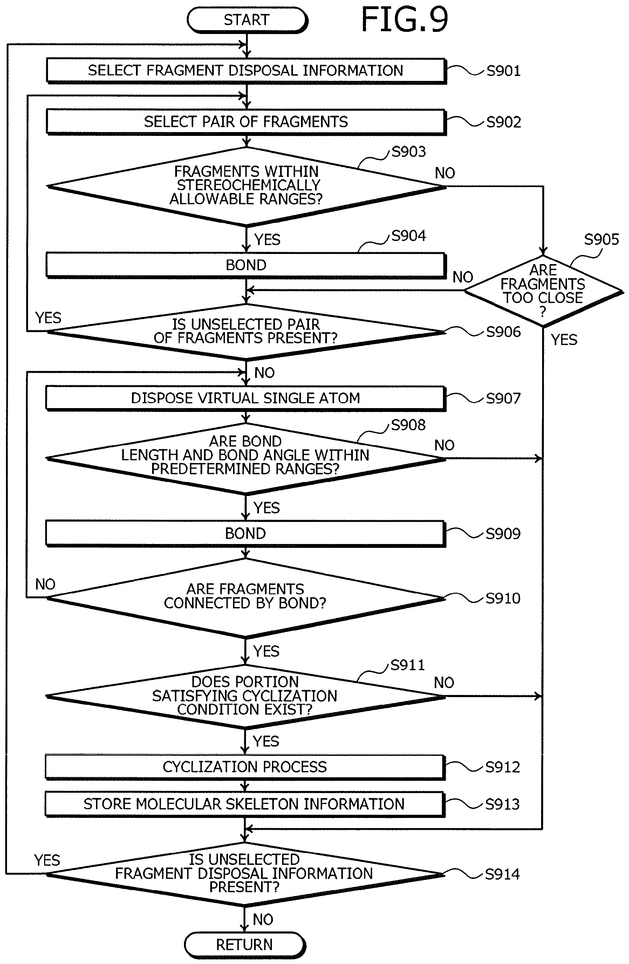

The compound design apparatus 100 executes a molecular skeleton construction process (step S604). The molecular skeleton construction process is a process of constructing a molecular skeleton of a low-molecular compound interacting with the protein. A specific process procedure of the molecular skeleton construction process will be described later with reference to FIG. 9.

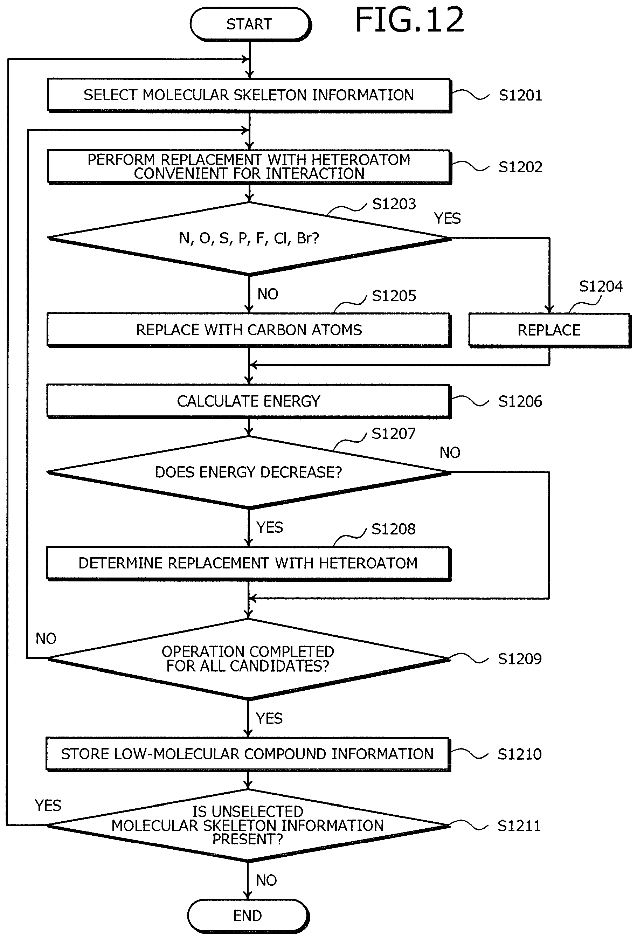

The compound design apparatus 100 executes a heteroatom replacement process (step S606) and terminates a series of operations according to the flowchart. The heteroatom replacement process is a process of replacing a virtual single atom with a heteroatom or a carbon atom. A specific process procedure of the heteroatom replacement process will be described later with reference to FIG. 12.

As a result, a low-molecular compound interacting with the protein can be designed.

<Fragment Selection Process Procedure>

A specific process procedure of the fragment selection process at step S602 depicted in FIG. 6 will be described.

FIG. 7 is a flowchart of an example of a specific process procedure of the fragment selection process. In the flowchart depicted in FIG. 7, first, based on the active site information 320 obtained at step S601 depicted in FIG. 6, the compound design apparatus 100 calculates an atom count of a cube that includes the active site of the protein disposed on the simulation space (step S701).

The compound design apparatus 100 selects a large fragment having an atom count equal to or greater than the predetermined number N or having a volume of the fragment equal to or greater than the predetermined volume V (step S702). The compound design apparatus 100 determines whether the rate of the total number of atoms in the selected large fragment to the calculated atom count reaches a rate of about 30 percent (step S703).

If a rate of about 30 percent is not reached (step S703: NO), the compound design apparatus 100 returns to step S702. On the other hand, if a rate of about 30 percent is reached (step S703: YES), the compound design apparatus 100 selects a small fragment having an atom count less than the predetermined number N or having a volume of the fragment less than the predetermined volume V (step S704) .

The compound design apparatus 100 determines whether a rate of the total number of atoms in the selected large and small fragments to the calculated atom count reaches a rate of about 50 percent (step S705). If a rate of about 50 percent is not reached (step S705: NO), the compound design apparatus 100 returns to step S704.

On the other hand, if a rate of about 50 percent is reached (step S705: YES), the compound design apparatus 100 stores the selected large and small fragments as a segment set (step S706). The compound design apparatus 100 determines whether a preset predetermined number of segment sets is stored (step S707).

If the predetermined number of segment sets is not stored (step S707: NO), the compound design apparatus 100 returns to step S702. On the other hand, if the predetermined number of segment sets is stored (step S707: YES), the compound design apparatus 100 terminates a series of processes of this flowchart and returns to the step at which the fragment selection process is called.

As a result, the fragment set of fragments to be disposed can be selected. It is noted that 30 percent and 50 percent described above are examples and an optimum numerical value may be selected depending on an active site status or type of fragments to be introduced.

Although a standard initial filling amount is, for example, 180 atoms/nm$^3$ in the case of diamond-type closest packing (density of 3.15 g/cm$^3$), the closest packing is too packed and the initial filling amount may be set within a range of 30 to 90 atoms/nm$^3$. Since an active site space is about 0.8 nm$^3$ in an example of an HIV protease inhibitor (PDB: 1D4H) and the inhibitor has 44 atoms (excluding hydrogen), 55 atoms/nm$^3$ results and this level is considered as a standard for a large number of drugs (final filling). The initial filling in the present embodiment is made smaller to about 20 to 40 atoms/nm$^3$

and virtual single atoms are packed from this level.

<Fragment Disposal Process Procedure>

A specific process procedure of the fragment disposal process of step S603 depicted in FIG. 6 will be described.

FIG. 8 is a flowchart of an example of a specific process procedure of the fragment disposal process. In the flowchart depicted in FIG. 8, first, the compound design apparatus 100 extracts a fragment set from among the predetermined number of fragment sets stored at step S706 depicted in FIG. 7 (step S801).

Based on the conformation information 310 and the active site information 320 of the protein, the compound design apparatus 100 randomly disposes at the active site of the protein disposed on the simulation space, the fragments (large fragments and small fragments) included in the extracted fragment set (step S802).

The compound design apparatus 100 performs MD calculation based on interaction between the protein and the fragments and interaction between the fragments (step S803). The compound design apparatus 100 evaluates only the interaction between the protein and the fragments to determine whether the interaction is higher than a predetermined reference (step S804).

If the interaction is lower than the predetermined reference (step S804: NO), the compound design apparatus 100 goes to step S806. On the other hand, if the interaction is higher than the predetermined reference (step S804: YES), the compound design apparatus 100 stores fragment disposal information (step S805). The fragment disposal information is information representative of the fragments disposed at the active site of the protein disposed on the simulation space.

The compound design apparatus 100 determines whether a fragment set exists remains that has not been extracted among the predetermined number of the fragment sets (step S806). If a fragment set remains (step S806: YES), the compound design apparatus 100 returns to step S801, extracts a fragment set from among the predetermined number of the fragment sets.

On the other hand, if no fragment set remains (step S806: NO), the compound design apparatus 100 terminates a series of operations according to the flowchart and returns to the step at which the fragment disposal process is called.

As a result, a disposal result can be obtained where the fragments are disposed in a region relatively close to an inner wall of the protein. If a calculation time is short, all the possibilities cannot be exploited and a result may be dependent on initial disposal (relative positions of fragments, directions of fragments) into the active site of the protein. Therefore, the compound design apparatus 100 may perform the MD calculation when the initial disposal patterns differ.

Although a case of executing the fragment disposal process after storing the predetermined number of fragment sets is taken as an example in the above description, this is not a limitation. For example, the compound design apparatus 100 may execute the fragment disposal process each time a fragment set is stored.

<Molecular Skeleton Construction Process Procedure>

A specific process procedure of the molecular skeleton construction process of step S604 depicted in FIG. 6 will be described.

FIG. 9 is a flowchart of an example of a specific process procedure of the molecular skeleton construction process. In the flowchart depicted in FIG. 9, the compound design apparatus 100 selects the fragment disposal information stored at step S805 depicted in FIG. 8 (step S901) .

The compound design apparatus 100 refers to the selected fragment disposal information and selects a pair of fragments disposed on the simulation space (step S902). The compound design apparatus 100 determines whether the selected fragments have a bond length and a bond angle to each other within stereochemically allowable ranges (step S903) .

If within the stereochemically allowable ranges (step S903: YES), the compound design apparatus 100 bonds the fragments within the stereochemically allowable ranges (step S904) and goes to step S906. If not within the stereochemically allowable ranges (step S903: NO), the compound design apparatus 100 determines whether the fragments are too close to each other (step S905). For example, if a shortest interatomic distance between the fragments is within a predetermined distance, the compound design apparatus 100 determines that the fragments are too close to each other.

If the fragments are too close to each other (step S905: YES), the compound design apparatus 100 excludes the selected fragment disposal information from candidates and goes to step S914. On the other hand, if the fragments are not too close to each other (step S905: NO), the compound design apparatus 100 goes to step S906.

The compound design apparatus 100 determines whether a pair of fragments remains that has not been selected among the fragments disposed on the simulation space (step S906). If a pair of fragments remains (step S906: YES), the compound design apparatus 100 returns to step S902 and selects a pair of fragments disposed on the simulation space.

On the other hand, if no unselected pair of fragments remains (step S906: NO), the compound design apparatus 100 disposes a virtual single atom at a position that does not collide with the protein, the fragments, or the virtual single atoms disposed on the simulation space (step S907). In particular, since gaps are present everywhere when only the fragments are disposed, the compound design apparatus 100 introduces virtual single atoms onto the simulation space so as to fill the gaps and searches for an arrangement that can be expected to achieve the bondability.

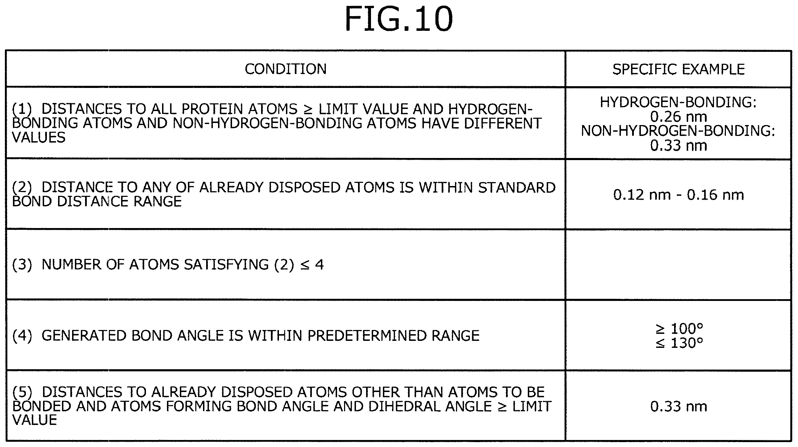

The compound design apparatus 100 then determines whether the bond length and the bond angle formed between the disposed virtual single atom and an atom (virtual single atom) of an already disposed fragment or an already disposed virtual single atom are within predetermined ranges (step S908). Whether the bond length and the bond angle are within predetermined ranges is determined by the compound design apparatus 100, for example, based on a condition of whether virtual atom arrangement is possible as depicted in FIG. 10 described later.

If the bond length and the bond angle are not within the predetermined ranges (step S908: NO), the compound design apparatus 100 goes to step S914. On the other hand, if the bond length and the bond angle are within the predetermined ranges (step S908: YES), the compound design apparatus 100 forms a bond to the disposed virtual single atom (step S909).

The compound design apparatus 100 determines whether fragments are connected to each other by the bond (step S910). If fragments are not connected to each other by the bond (step S910: NO), the compound design apparatus 100 returns to step S907. On the other hand, if fragments are connected to each other by the bond (step S910: YES), the compound design apparatus 100 determines whether a portion satisfying the cyclization condition exists on the molecular skeleton of the fragments connected to each other by the bond (step S911).

If a portion satisfying the cyclization condition does not exist (step S911: NO), the compound design apparatus 100 goes to step S914. On the other hand, if a portion satisfying the cyclization condition exists (step S911: YES), the compound design apparatus 100 executes the cyclization process of the portion satisfying the cyclization condition (step S912) and stores the molecular skeleton information (step S913).

The compound design apparatus 100 determines whether unselected fragment disposal information is present (step S914). If unselected fragment disposal information is present (step S914: YES), the compound design apparatus 100 returns to step S901 and selects unselected fragment disposal information.

On the other hand, if no unselected fragment disposal information is present (step S914: NO), the compound design apparatus 100 terminates a series of operations according to the flowchart and returns to the step at which the molecular skeleton construction process is called.

As a result, a molecular skeleton of a low-molecular compound having fragments connected to each other by a linker that includes a ring structure can be constructed. The condition concerning whether virtual atom arrangement is possible used when determining whether the bond length and the bond angle are within the predetermined angles at step S908 described above.

FIG. 10 is an explanatory view of an example of the condition concerning whether virtual atom arrangement is possible. In the example depicted in FIG. 10, the compound design apparatus 100 defines the bond length as 0.12 mm to 0.16 nm and the bond angle as 100 degrees to 130 degrees, and searches for all bondable counterpart atoms within these ranges. With regard to the bond angle, the compound design apparatus 100 also examines whether a bond angle formed by a newly formed bond and an originally existing bond beyond the bond is within the allowable range.

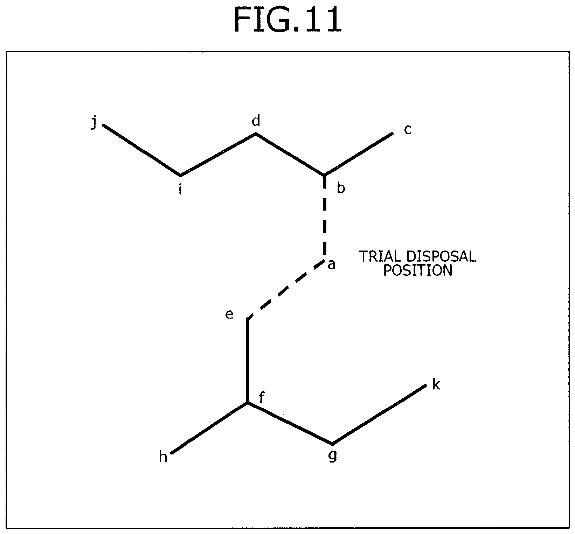

FIG. 11 is an explanatory view of details of a condition (5) depicted in FIG. 10. In FIG. 11, when a trial disposal position is a, the condition (5) represents that Rab and Rae are within the allowable range, that $\theta$bae, $\theta$abc, $\theta$abd, and $\theta$aef are within the allowable range and that Daj, Dak, etc. are equal to or greater than a limit distance (within the allowable range). Rab, Rae, Daj, and Dak indicate distances between a and b, a and e, a and j, and a and k, respectively, and $\theta$bae, $\theta$abc, $\theta$abd, and $\theta$aef indicate angles formed between ba and ea, ab and cb, ab and db, and ae and fe, respectively.

A specific process procedure of the heteroatom replacement process of step S605 depicted in FIG. 6 will be described.

FIG. 12 is a flowchart of an example of a specific process procedure of the heteroatom replacement process. In the flowchart of FIG. 12, first, the compound design apparatus 100 selects the molecular skeleton information stored at step S913 depicted in FIG. 9 (step S1201).

The compound design apparatus 100 refers to the selected molecular skeleton information to perform replacement with a heteroatom convenient for interaction based on correlation with an amino acid on the protein side (step S1202). For example, the compound design apparatus 100 disposes a negative electric charge for a positive electric charge or vice versa and disposes an atom acting as a hydrogen-bond acceptor for an atom acting as a hydrogen-bond donor or vice versa.

If N, O, S, P, F, Cl, Br, etc. are replacement candidates (step S1203: YES), the compound design apparatus 100 performs replacement without change (step S1204) and replaces others (step S1203: NO) with carbon atoms (step S1205). The heteroatom replacement normally has possibilities of a multiplicity of candidates.

The compound design apparatus 100 takes one given portion to calculate an electrostatic interaction energy value for determining whether the given heteroatom replacement is effective (step S1206). The compound design apparatus 100 makes an evaluation based on increase/decrease in the calculated energy value and, if advantageous, i.e., if the energy decreases (step S1207: YES), the compound design apparatus 100 adopts and determines the replacement with the heteroatom (step S1208).

On the other hand, if disadvantageous (step S1207: NO), the compound design apparatus 100 does not adopt the replacement and goes to step S1209. Subsequently, the compound design apparatus 100 continues the same operation for another place (step S1209: NO) and when the operation is completed for all the candidates (step S1209: YES), the compound design apparatus 100 stores the low-molecular compound information (step S1210).

The compound design apparatus 100 determines whether unselected molecular skeleton information is present (step S1211). If unselected molecular skeleton information is present (step S1211: YES), the compound design apparatus 100 returns to step S1201 and selects unselected molecular skeleton information. On the other hand, if no unselected molecular skeleton information is present (step S1211: NO), the compound design apparatus 100 terminates a series of operations according to the flowchart.

As a result, a candidate structure of a low-molecular compound can be determined. Since a dihedral angle has not been considered up to this point, the compound design apparatus 100 excludes those including an abnormal dihedral angle. To evaluate overall docking with the protein, the compound design apparatus 100 calculates and confirms a bond free energy value. The compound design apparatus 100 uses, for example, an index of drug-likeness such as Lipinski's Rule for final confirmation and excludes those that cannot be expected as a candidate. In this way, the structure of the final candidate can be determined.

(Construction Example of Molecular Skeleton of Low-Molecular Compound)

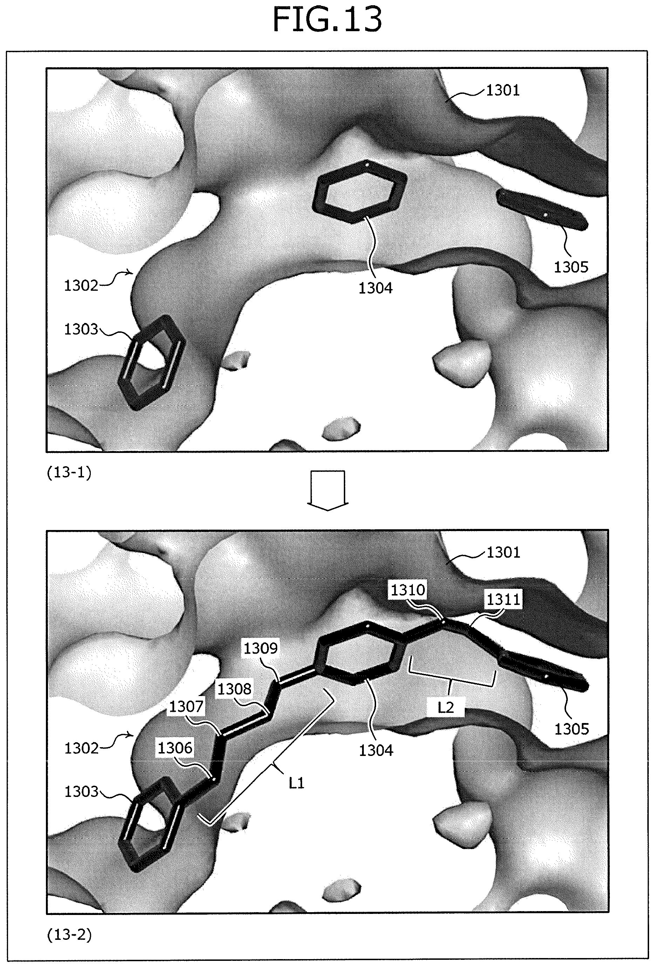

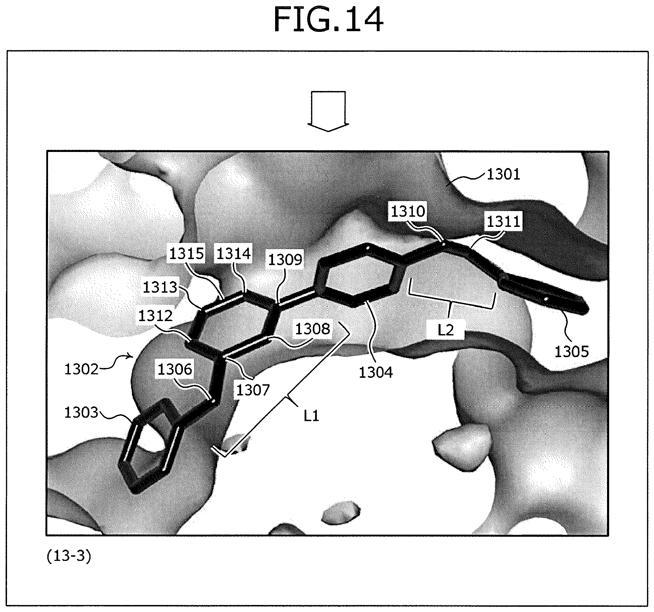

FIGs. 13 and 14 are explanatory views of a construction example of a molecular skeleton of a low-molecular compound. In (13-1) of FIG. 13, fragments 1303 to 1305 are disposed at an active site 1302 of a protein 1301 on a simulation space.

In (13-2) of FIG. 13, as a result of disposing virtual single atoms 1306 to 1311 at positions that do not collide with the protein 1301 and the fragments 1303 to 1305 for the active site 1302 of the protein 1301, the fragments are bonded with each other. For example, the fragment 1303 and the fragment 1304 are connected by a linker L1. The fragment 1304 and the fragment 1305 are connected by a linker L2.

In (13-3) of FIG. 14, as a result of adding virtual single atoms 1312 to 1314 to contiguous virtual single atoms 1307 to 1309 satisfying the cyclization condition, a ring 1315 is generated on the linker L1. As a result, a molecular skeleton of a low-molecular compound is constructed that has the fragments 1303, 1304 connected to each other by the linker L1 that includes the ring 1315.

As described above, the compound design apparatus 100 according to the present embodiment can dispose a virtual single atom at a position that does not collide with a protein, a fragment, or a virtual single atom disposed on a simulation space and can bond fragments with each other, a fragment with a virtual single atom, and virtual single atoms with each other when bondability exists. This enables the construction of a molecular skeleton of a low-molecular compound that interacts with the protein.

The compound design apparatus 100 can execute the cyclization process when a set of three contiguous atoms including a virtual single atom forming a bond satisfies the cyclization condition under which a chemically proper ring may be formed if a virtual single atom is added to these atoms. This enables the construction of a molecular skeleton of a low-molecular compound having fragments connected to each other by a linker that includes a ring.

The compound design apparatus 100 can execute the cyclization process if an angle formed between a first vector from a first atom to a second atom and a second vector from the second atom to a third atom in the set of the three atoms is the predetermined angle $\alpha$. This enables the generation of a chemically proper ring on the linker connecting the fragments to each other.

The compound design apparatus 100 can terminate the cyclization process if the number of virtual single atoms in a portion connecting the generated ring to the fragment and/or a portion connecting the generated rings to each other is equal to or less than the predetermined number $\beta$. Therefore, the cyclization process can be terminated if a degree of freedom of atoms in a low-molecular compound is reduced to a level facilitating an interaction with protein.

Thus, the compound design apparatus 100 can efficiently design a low-molecular compound interacting with a protein having a known three-dimensional structure. For example, the compound design apparatus 100 can increase the probability that a linker that includes a ring structure is generated as a linker connecting fragments of a low-molecular compound to each other, thereby improving calculation efficiency when designing a low-molecular compound producing an advantageous interaction with the protein.

The compound design method described in the present embodiment may be implemented by executing a prepared program on a computer such as a personal computer and a workstation. The program is stored on a computer-readable recording medium such as a hard disk, a flexible disk, a CD-ROM, an MO, and a DVD, read out from the computer-readable medium, and executed by the computer. The program may be distributed through a network such as the Internet.

EXPLANATIONS OF LETTERS OR NUMERALS

100    compound design apparatus
110    fragment library
301    obtaining unit
302    selecting unit
303    disposing unit
304    first determining unit
305    bonding unit
306    second determining unit
307    cyclization process unit
308    replacing unit
309    output unit

**Claims**

1. A compound design program causing a computer to execute a process comprising:

   disposing a virtual single atom at a position that does not collide with a protein, a fragment, or a virtual single atom disposed on a simulation space;
   forming a bond when an interatomic distance and an angle formed by the disposed virtual single atom with an atom in the fragment or another virtual single atom are within predetermined ranges for bond length and bond angle;
   determining whether an angle formed between a first vector from a first atom to a second atom and a second vector from the second atom to a third atom in a set of three contiguous atoms including the disposed virtual single atom is a predetermined angle; and
   executing a cyclization process of generating a ring by adding a virtual single atom to the set of three atoms determined when the angle formed between the first vector and the second vector is determined to be the predetermined angle.

2. The compound design program according to claim 1, wherein
   the executing of the cyclization process includes generating a ring by disposing a virtual single atom at a position at which the chemically proper ring may be formed with the three atoms and forming bonds with the three atoms.

3. The compound design program according to claim 2, the process further comprising
   terminating the executing of the cyclization process when a count of virtual single atoms in a portion connecting the generated ring to the fragment and/or a portion connecting the generated rings to each other is equal to or less than a predetermined number.

4. A compound design apparatus comprising:

   a disposing unit configured to dispose a virtual single atom at a position that does not collide with a protein, a fragment, or a virtual single atom disposed on a simulation space;
   a bonding unit configured to form a bond when an interatomic distance and an angle formed by the virtual single atom disposed by the disposing unit with an atom in the fragment or another virtual single atom are within predetermined ranges for bond length and bond angle;

a determining unit configured to determine whether an angle formed between a first vector from a first atom to a second atom and a second vector from the second atom to a third atom in a set of three contiguous atoms including the disposed virtual single atom is a predetermined angle; and

a processing unit configured to execute a cyclization process of generating a ring by adding a virtual single atom to the set of three atoms determined when the angle formed between the first vector and the second vector is determined to be the predetermined angle.

5. A compound design method comprising:

disposing a virtual single atom at a position that does not collide with a protein, a fragment, or a virtual single atom disposed on a simulation space;

forming a bond when an interatomic distance and an angle formed by the disposed virtual single atom with an atom in the fragment or another virtual single atom are within predetermined ranges for bond length and bond angle;

determining whether an angle formed between a first vector from a first atom to a second atom and a second vector from the second atom to a third atom in a set of three contiguous atoms including the disposed virtual single atom is a predetermined angle; and

executing a cyclization process of generating a ring by adding a virtual single atom to the set of three atoms determined when the angle formed between the first vector and the second vector is determined to be the predetermined angle, wherein

the compound design method is executed by a computer.

**Patentansprüche**

1. Programm für die Konstruktion einer Verbindung, das einen Computer veranlasst, einen Prozess auszuführen, umfassend:

Anordnen eines virtuellen Einzelatoms an einer Position, die nicht mit einem Protein, einem Fragment oder einem virtuellen Einzelatom auf einem Simulationsraum kollidiert;

Bilden einer Bindung, wenn ein interatomarer Abstand und ein Winkel, der durch das angeordnete virtuelle Einzelatom mit einem Atom im Fragment oder einem anderen virtuellen Einzelatom gebildet wird, innerhalb vorgegebener Bereiche für Bindungslänge und Bindungswinkel liegen;

Bestimmen, ob ein Winkel, der zwischen einem ersten Vektor von einem ersten Atom zu einem zweiten Atom und einem zweiten Vektor von dem zweiten Atom zu einem dritten Atom in einem Satz von drei benachbarten Atomen einschließlich des angeordneten virtuellen Einzelatoms gebildet wird, ein vorbestimmter Winkel ist; und

Ausführen eines Zyklisierungsprozesses zum Erzeugen eines Rings durch Hinzufügen eines virtuellen Einzelatoms zu dem Satz von drei Atomen, der bestimmt wird, wenn der zwischen dem ersten Vektor und dem zweiten Vektor gebildete Winkel als der vorbestimmte Winkel bestimmt wird.

2. Programm für die Konstruktion einer Verbindung nach Anspruch 1, wobei
die Ausführung des Zyklisierungsprozesses ein Erzeugen eines Rings durch Anordnen eines virtuellen Einzelatoms an einer Position, an der der chemisch ordnungsgemäße Ring mit den drei Atomen gebildet werden kann, und das Bilden von Bindungen mit den drei Atomen beinhaltet.

3. Programm für die Konstruktion einer Verbindung nach Anspruch 2, der Prozess weiter umfassend
ein Beenden der Ausführung des Zyklisierungsprozesses, wenn eine Zahl virtueller Einzelatome in einem Abschnitt, der den erzeugten Ring mit dem Fragment verbindet, und/oder einem Abschnitt, der die erzeugten Ringe miteinander verbindet, gleich oder kleiner als eine vorbestimmte Anzahl ist.

4. Einrichtung für die Konstruktion einer Verbindung, umfassend:

eine Anordnungseinheit, die konfiguriert ist, um ein virtuelles Einzelatom an einer Position anzuordnen, die nicht mit einem Protein, einem Fragment oder einem virtuellen Einzelatom auf einem Simulationsraum kollidiert;

eine Bindungseinheit, die konfiguriert ist, um eine Bindung zu bilden, wenn ein interatomarer Abstand und ein Winkel, der durch das von der Anordnungseinheit angeordnete virtuelle Einzelatom mit einem Atom im Fragment oder einem anderen virtuellen Einzelatom gebildet wird, innerhalb vorgegebener Bereiche für Bindungslänge und Bindungswinkel liegen;

eine Bestimmungseinheit, die konfiguriert ist, um zu bestimmen, ob ein Winkel, der zwischen einem ersten Vektor von einem ersten Atom zu einem zweiten Atom und einem zweiten Vektor von dem zweiten Atom zu einem dritten Atom in einem Satz von drei benachbarten Atomen einschließlich des angeordneten virtuellen Einzelatoms gebildet wird, ein vorbestimmter Winkel ist; und

eine Verarbeitungseinheit, die konfiguriert ist, um einen Zyklisierungsprozess zum Erzeugen eines Rings durch Hinzufügen eines virtuellen Einzelatoms zu dem Satz von drei Atomen auszuführen, der bestimmt wird, wenn der zwischen dem ersten Vektor und dem zweiten Vektor gebildete Winkel als der vorbestimmte Winkel bestimmt wird.

5. Verfahren für die Konstruktion einer Verbindung, umfassend:

Anordnen eines virtuellen Einzelatoms an einer Position, die nicht mit einem Protein, einem Fragment oder einem virtuellen Einzelatom auf einem Simulationsraum kollidiert;

Bilden einer Bindung, wenn ein interatomarer Abstand und ein Winkel, der durch das angeordnete virtuelle Einzelatom mit einem Atom im Fragment oder einem anderen virtuellen Einzelatom gebildet wird, innerhalb vorgegebener Bereiche für Bindungslänge und Bindungswinkel liegen;

Bestimmen, ob ein Winkel, der zwischen einem ersten Vektor von einem ersten Atom zu einem zweiten Atom und einem zweiten Vektor von dem zweiten Atom zu einem dritten Atom in einem Satz von drei benachbarten Atomen einschließlich des angeordneten virtuellen Einzelatoms gebildet wird, ein vorbestimmter Winkel ist; und

Ausführen eines Zyklisierungsprozesses zum Erzeugen eines Rings durch Hinzufügen eines virtuellen Einzelatoms zu dem Satz von drei Atomen, der bestimmt wird, wenn der zwischen dem ersten Vektor und dem zweiten Vektor gebildete Winkel als der vorbestimmte Winkel bestimmt wird, wobei

das Verfahren für die Konstruktion einer Verbindung durch einen Computer ausgeführt wird.

**Revendications**

1. Programme de conception d'un composé amenant un ordinateur à exécuter un processus comprenant les étapes consistant à :

disposer un atome virtuel unique dans une position qui n'entre pas en collision avec une protéine, un fragment ou un atome virtuel unique disposé(e) sur un espace de simulation ;

former une liaison lorsqu'une distance interatomique et un angle formés par l'atome virtuel unique disposé avec un atome dans le fragment ou un autre atome virtuel unique se situent dans des plages prédéterminées pour la longueur de liaison et l'angle de liaison ;

déterminer si un angle formé entre un premier vecteur d'un premier atome à un deuxième atome et un second vecteur du deuxième atome à un troisième atome dans un ensemble de trois atomes contigus incluant l'atome virtuel unique disposé est un angle prédéterminé ; et

exécuter un processus de cyclisation consistant à générer un cycle en ajoutant un atome virtuel unique à l'ensemble de trois atomes déterminé lorsque l'angle formé entre le premier vecteur et le second vecteur est déterminé comme étant l'angle prédéterminé.

2. Programme de conception d'un composé selon la revendication 1, dans lequel

l'exécution du processus de cyclisation inclut la génération d'un cycle en disposant un atome virtuel unique dans une position dans laquelle le cycle chimiquement correct peut être formé avec les trois atomes et la formation de liaisons avec les trois atomes.

3. Programme de conception d'un composé selon la revendication 2, le procédé comprenant en outre l'étape consistant à

mettre fin à l'exécution du processus de cyclisation lorsqu'un décompte d'atomes virtuels uniques dans une partie reliant le cycle généré au fragment et/ou une partie reliant les cycles générés les uns aux autres est égal ou inférieur à un nombre prédéterminé.

4. Appareil de conception d'un composé comprenant :

une unité de disposition configurée pour disposer un atome virtuel unique dans une position qui n'entre pas en collision avec une protéine, un fragment ou un atome virtuel unique disposé(e) sur un espace de simulation ;

une unité de liaison configurée pour former une liaison lorsqu'une distance interatomique et un angle formés par l'atome virtuel unique disposé par l'unité de disposition avec un atome dans le fragment ou un autre atome virtuel unique se situent dans des plages prédéterminées pour la longueur de liaison et l'angle de liaison ;

une unité de détermination configurée pour déterminer si un angle formé entre un premier vecteur d'un premier atome à un deuxième atome et un second vecteur du deuxième atome à un troisième atome dans un ensemble de trois atomes contigus incluant l'atome virtuel unique disposé est un angle prédéterminé ; et

une unité de traitement configurée pour exécuter un processus de cyclisation consistant à générer un cycle en ajoutant un atome virtuel unique à l'ensemble de trois atomes déterminé lorsque l'angle formé entre le premier vecteur et le second vecteur est déterminé comme étant l'angle prédéterminé.

5. Procédé de conception d'un composé, comprenant les étapes consistant à :

disposer un atome virtuel unique dans une position qui n'entre pas en collision avec une protéine, un fragment ou un atome virtuel unique disposé(e) sur un espace de simulation ;
former une liaison lorsqu'une distance interatomique et un angle formés par l'atome virtuel unique disposé avec un atome dans le fragment ou un autre atome virtuel unique se situent dans des plages prédéterminées pour la longueur de liaison et l'angle de liaison ;
déterminer si un angle formé entre un premier vecteur d'un premier atome à un deuxième atome et un second vecteur du deuxième atome à un troisième atome dans un ensemble de trois atomes contigus incluant l'atome virtuel unique disposé est un angle prédéterminé ; et
exécuter un processus de cyclisation consistant à générer un cycle en ajoutant un atome virtuel unique à l'ensemble de trois atomes déterminé lorsque l'angle formé entre le premier vecteur et le second vecteur est déterminé comme étant l'angle prédéterminé, dans lequel
le procédé de conception d'un composé est exécuté par un ordinateur.

# FIG.1

EP 2 977 923 B1

# FIG.2

EP 2 977 923 B1

# FIG.3

100

FRAGMENT LIBRARY ~110

302 303 304 305 306 307 308

| SELECTING UNIT | DISPOSING UNIT | FIRST DETERMIN-ING UNIT | BONDING UNIT | SECOND DETERMIN-ING UNIT | CYCLIZATION PROCESS UNIT | REPLACING UNIT |

OBTAINING UNIT ~301

OUTPUT UNIT

309

CONFORMATION INFORMATION

ACTIVE SITE INFORMATION

310 320

EP 2 977 923 B1

# FIG.4

# FIG.5

FRAGMENT

VIRTUAL SINGLE ATOM AND NORMAL VECTOR

# FIG.6

START

OBTAIN CONFORMATION INFORMATION AND ACTIVE SITE INFORMATION OF PROTEIN — S601

FRAGMENT SELECTION PROCESS — S602

FRAGMENT DISPOSAL PROCESS — S603

SKELETON CONSTRUCTION PROCESS — S604

HETEROATOM REPLACEMENT PROCESS — S605

END

# FIG.7

```
                    START

                       │
                       ▼
        ┌──────────────────────────────┐
        │    CALCULATE ATOM COUNT       │──── S701
        └──────────────────────────────┘
                       │
      ┌───────────────▶│
      │                ▼
      │  ┌──────────────────────────────┐
      │  │    SELECT LARGE FRAGMENT      │──── S702
      │  └──────────────────────────────┘
      │                │
      │                ▼
   NO │        ◇──────────────────◇
      │◀───────  IS RATE ABOUT 30%?  ──── S703
      │        ◇──────────────────◇
      │                │
      │              YES
      │                ▼
      │    ┌──────────────────────────────┐
      │    │    SELECT SMALL FRAGMENT      │──── S704
      │    └──────────────────────────────┘
      │                │
   NO │                ▼
      │        ◇──────────────────◇
      │◀───────  IS RATE ABOUT 50%?  ──── S705
      │        ◇──────────────────◇
      │                │
      │              YES
      │                ▼
      │    ┌──────────────────────────────┐
      │    │      STORE AS SEGMENT         │──── S706
      │    └──────────────────────────────┘
      │                │
   NO │                ▼
      │        ◇──────────────────────────◇
      └────────  IS PREDETERMINED NUMBER    ──── S707
               OF SEGMENT SETS STORED?
               ◇──────────────────────────◇
                       │
                     YES
                       ▼
                    RETURN
```

# FIG.8

START

EXTRACT FRAGMENT SET — S801

DISPOSE FRAGMENTS — S802

EXECUTE MD CALCULATION — S803

IS PROTEIN AND FRAGMENT INTERACTION HIGH? — S804

NO

YES

STORE FRAGMENT DISPOSAL INFORMATION — S805

IS UNEXTRACTED FRAGMENT SET PRESENT? — S806

YES

NO

RETURN

# FIG.9

```
        START
          │
          ▼
SELECT FRAGMENT DISPOSAL INFORMATION ──── S901
          │
          ▼
   SELECT PAIR OF FRAGMENTS ──── S902
          │
          ▼
      FRAGMENTS WITHIN                 S903
      STEREOCHEMICALLY ─────── NO ──────┐
     ALLOWABLE RANGES?                  │
          │ YES                         │
          ▼          S904               ▼
        BOND                     ARE           S905
          │              NO ── FRAGMENTS
          ▼            ◄────── TOO CLOSE
   IS UNSELECTED PAIR              ?
YES─ OF FRAGMENTS PRESENT? ─ S906     │ YES
          │ NO
          ▼
  DISPOSE VIRTUAL SINGLE ATOM ──── S907
          │
          ▼
        ARE BOND                     S908
   LENGTH AND BOND ANGLE WITHIN ── NO ──►
    PREDETERMINED RANGES?
          │ YES
          ▼
        BOND ──── S909
          │
          ▼
      ARE FRAGMENTS
NO ─ CONNECTED BY BOND? ──── S910
          │ YES
          ▼
      DOES PORTION              S911
  SATISFYING CYCLIZATION ── NO ──►
    CONDITION EXIST?
          │ YES
          ▼
   CYCLIZATION PROCESS ──── S912
          │
          ▼
STORE MOLECULAR SKELETON INFORMATION ──── S913
          │
          ▼
      IS UNSELECTED
YES ─ FRAGMENT DISPOSAL INFORMATION ──── S914
        PRESENT?
          │ NO
          ▼
        RETURN
```

# FIG.10

| CONDITION | SPECIFIC EXAMPLE |
|---|---|
| (1) DISTANCES TO ALL PROTEIN ATOMS $\geq$ LIMIT VALUE AND HYDROGEN-BONDING ATOMS AND NON-HYDROGEN-BONDING ATOMS HAVE DIFFERENT VALUES | HYDROGEN-BONDING: 0.26 nm<br>NON-HYDROGEN-BONDING: 0.33 nm |
| (2) DISTANCE TO ANY OF ALREADY DISPOSED ATOMS IS WITHIN STANDARD BOND DISTANCE RANGE | 0.12 nm - 0.16 nm |
| (3) NUMBER OF ATOMS SATISFYING (2) $\leq$ 4 | |
| (4) GENERATED BOND ANGLE IS WITHIN PREDETERMINED RANGE | $\geq 100°$<br>$\leq 130°$ |
| (5) DISTANCES TO ALREADY DISPOSED ATOMS OTHER THAN ATOMS TO BE BONDED AND ATOMS FORMING BOND ANGLE AND DIHEDRAL ANGLE $\geq$ LIMIT VALUE | 0.33 nm |

# FIG.11

TRIAL DISPOSAL
POSITION

# FIG.12

```
                        START

                          │
                          ▼
        ┌─────────────────────────────────────┐
        │ SELECT MOLECULAR SKELETON INFORMATION │──── S1201
        └─────────────────────────────────────┘
                          │
                          ▼
        ┌─────────────────────────────────────┐
        │ PERFORM REPLACEMENT WITH HETEROATOM  │──── S1202
        │      CONVENIENT FOR INTERACTION      │
        └─────────────────────────────────────┘
                          │
                          ▼            S1203        YES
                   ◇─────────────────────────◇──────────┐
                   │   N, O, S, P, F, Cl, Br?  │          │
                   ◇─────────────────────────◇          │
                          │ NO      S1205               S1204
                          ▼                              ▼
        ┌─────────────────────────────┐        ┌──────────────┐
        │   REPLACE WITH CARBON ATOMS  │        │   REPLACE    │
        └─────────────────────────────┘        └──────────────┘
                          │                              │
                          ▼◄─────────────────────────────┘
        ┌─────────────────────────────┐
        │       CALCULATE ENERGY       │──── S1206
        └─────────────────────────────┘
                          │
                          ▼        S1207          NO
                   ◇─────────────────────◇──────────────┐
                   │  DOES ENERGY DECREASE? │             │
                   ◇─────────────────────◇             │
                          │ YES     S1208               │
                          ▼                             │
        ┌───────────────────────────────────────┐      │
        │ DETERMINE REPLACEMENT WITH HETEROATOM  │      │
        └───────────────────────────────────────┘      │
                          │◄──────────────────────────────┘
       NO                 ▼
    ◄───────────◇──────────────────────────◇──── S1209
                │  OPERATION COMPLETED        │
                │  FOR ALL CANDIDATES?        │
                ◇──────────────────────────◇
                          │ YES
                          ▼
        ┌─────────────────────────────────────────┐
        │ STORE LOW-MOLECULAR COMPOUND INFORMATION │──── S1210
        └─────────────────────────────────────────┘
                          │
      YES                 ▼
    ◄───────────◇──────────────────────────◇──── S1211
                │     IS UNSELECTED           │
                │ MOLECULAR SKELETON INFORMATION │
                │        PRESENT?             │
                ◇──────────────────────────◇
                          │ NO
                          ▼
                        END
```

# FIG.13

(13-1)

(13-2)

# FIG.14

(13-3)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005187374 A

**Non-patent literature cited in the description**

- GENSTAR: A METHOD FOR DE NOVO DRUG DE-SIGN. **ROTSTEIN S.H. et al.** JOURNAL OF COM-PUTER-AIDED MOLECULAR DESIGN. ESCOM SCIENCE PUBLISHERS BV, vol. 7
- RASSE: a new method for structure-based drug de-sign. **LUO Z. et al.** JOURNAL OF CHEMICAL IN-FORMATION AND COMPUTER SCIENCES. AMERICAN CHEMICAL SOCIETY, vol. 36
- AUTOMATIC CREATION OF DRUG CANDIDATE STRUCTURES BASED ON RECEPTOR STRUCTURE STARTING POINT FOR ARTIFICIAL LEAD GENERATION. **YOSHIHIKO NISHIBATA et al.** TET-RAHEDRON. ELSEVIER SCIENCE PUBLISHERS, vol. 47